# EUROPEAN PATENT APPLICATION

(11) **EP 1 642 892 A1**
(43) Date of publication of application: **05.04.2006**
(21) Application number: 04735103.6
(22) Date of filing: 27.05.2004
(51) Int. Cl.: C07D 211/46, C08L 101/00, C08K 5/3432

(54) **LACTONE-ADDED HINDERED AMINE COMPOUND**

(30) Priority: 28.05.2003 JP 2003150189
(71) Applicant: DAICEL CHEMICAL INDUSTRIES, LTD., Sakai-shi, Osaka 590-8501 (JP); Sankyo Lifetech Company, Limited, Tokyo 113-0033 (JP)
(72) Inventor: ENDO, Toshio, Hiroshima 739-0653 (JP); NAKAMOTO, Masanobu, Yamaguchi 7412-0061 (JP); TAKESHIBA, Hideo c/o Sankyo Co, Ltd., Tokyo 140-8710 (JP); YAMAZAKI, Takaaki c/o Sankyo Co, Ltd., Tokyo 140-8710 (JP); ICHIHASHI, Tetsuya c/o Sankyo Co, Ltd., Tokyo 140-8710 (JP)
(74) Representative: W.P. Thompson & Co.
(86) International application number: PCT/JP2004/007638
(87) International publication number: WO 2004/106303

(57) **Abstract**

The present invention provides a hindered amine compound that is suitable as a resin light stabilizer. Specifically, it provides a hindered amine compound represented by the following formula (1). 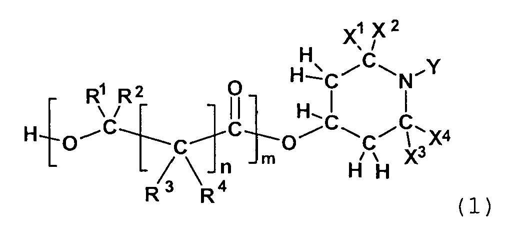 In the formula, R¹, R², R³ and R⁴ are each a hydrogen atom, a C₁ to C₁₀ substituted or unsubstituted alkyl group, etc.; X¹ to X⁴ are each a C₁ to C₁₀ substituted or unsubstituted alkyl group, etc.; Y is a hydrogen atom, an oxygen atom, a C₁ to C₁₀ substituted or unsubstituted alkyl group, etc.; n is an integer from 1 to 7; and m is an integer from 1 to 100.

## Description

### Technical Field to which the Invention Belongs

The present invention relates to a hindered amine compound, to a method for manufacturing a hindered amine compound, to a resin composition that yields a molded article with good weather resistance and contains a hindered amine compound, and to a molded article obtained from this resin composition.

### Background Art

With their high strength, durability, and good moldability, synthetic resins are commonly used in a wide range of industrial fields, such as automobiles, electrical and electronic products, and construction, but under actual usage conditions they have drawbacks in that they are degraded or discolored by heat, light, and nitrogen oxides (NOx), their molecular weight drops, and this in turn leads to a decrease in strength and also results in coloration.

Light stabilizers are sometimes added in an effort to improve the durability (weather resistance) of synthetic resins under actual usage conditions. Examples of such light stabilizers include bis(2,2,6,6-tetramethyl-4-piperidinyl) sebacate (trade name: Sanol®LS-770, made by Sankyo Lifetech) and bis(N,2,2,6,6-pentamethyl-4-piperidinyl) sebacate (trade name: Sanol®LS-292, made by Sankyo Lifetech). Anti-NOx agents such as HN130 and HN150 (made by Japan Hydrazine) have also been used to improve NOx resistance.

Nevertheless, because the above-mentioned conventional light stabilizers are low-boiling compounds with a low molecular weight, many different problems are encountered when these are added to a synthetic resin. For instance, when a large quantity of light stabilizer is added, it induces phase separation, which decreases the transparency and mechanical strength of the synthetic resin. In view of this, the amount of light stabilizers added is kept to a minimum, but this also prevents the weather resistance of the synthetic resin from being increased to a satisfactory level.

Also, low-molecular weight light stabilizers volatilize or are heat decomposed during the molding of the synthetic resin, or ooze from the surface of the molded article, and therefore tend to be a source of trouble during molding (such as mold fouling), and do not remain in the resin in a large enough amount to be effective, all of which makes it difficult to give a synthetic resin weather resistance that will remain stable over an extended period.

Attempts at solving the above problems have included raising the molecular weight of the light stabilizer to improve its compatibility with the resin and prevent the volatilization, heat decomposition, oozing, and so forth of the light stabilizer.

Known examples of this include a 2,4,4-trimethyl-2-pentanamine-modified 1,3,5-triazine copolymer of N,N'-bis(2,2,6,6-tetramethyl-4-piperidinyl)-1,6-hexanediamine (trade name: Sanol®LS-944, made by Sankyo Lifetech), a carboxylic acid-terminated methyl ester of a copolymer of succinic acid and N-(2-hydroxyethyl)-2,2,6,6-tetramethyl-4-hydroxypiperidine (trade name: Chisorb 622, made by Chitec), and N,N',N",N"'-tetrakis{2,6-bis[N-(N,2,2,6,6-pentamethyl-4-piperidinyl)butylamino]4-triazinyl}-N,N'-bis(3-aminopropyl)ethylenediamine (trade name: Chisorb 119, made by Chitec).

However, even these light stabilizers with increased molecular weight have the following drawbacks, and there is still room for improvement. Specifically, depending on the kind of synthetic resin, compatibility may be inadequate, which again lowers the mechanical strength of the synthetic resin. This tendency is particularly pronounced in thermoplastic resins such as polyolefins, polyvinyl chloride or polyvinylidene chloride. Furthermore, these light stabilizers are still not satisfactory in terms of long-term weather resistance.

Meanwhile, adding a semicarbazide compound and a hindered phenol-based antioxidant to a polyurethane has been proposed as a way to prevent discoloration or coloration of a polyurethane by NOx (JP-B 2,625,508). The problem, however, is that this does not adequately prevent discoloration and coloration. Further, a way of adding a hindered phenol-based antioxidant to a polyurethane has been proposed (JP-B 6-35538), but there is a problem that the effect of preventing discoloration or coloration by NOx was insufficient.

It is an object of the present invention to provide a hindered amine compound that has good compatibility with a wide range of synthetic resins when mixed with such resins, that creates no problems during resin molding, and that yields a molded resin with good weather resistance, as well as a method for manufacturing this hindered amine compound, a resin composition containing this hindered amine compound, and a molded article obtained from this resin composition.

### Disclosure of the Invention

The means in the present invention for solving the stated problems is to provide a hindered amine compound represented by the formula (1), a method for manufacturing this compound, a resin composition containing a hindered amine compound represented by the formula (1), and a molded article obtained from this resin composition. It is also an object to provide the use of this hindered amine compound as a resin additive. 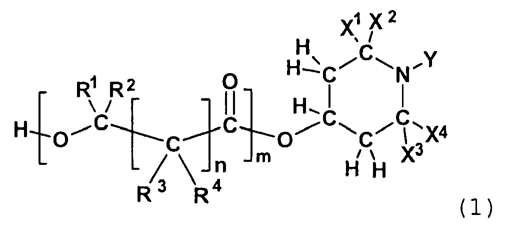 In the formula, the meanings of each symbols are as follows:
R¹, R², R³ and R⁴ are atoms or substituents selected from among a hydrogen atom, a C₁ to C₁₀ substituted or unsubstituted alkyl group and a substituted or unsubstituted aryl group, and R¹, R², R³ and R⁴ may be bound together to form one or more rings.
R³ and R⁴ may be the same as or different from each other, n-number of R³ and R⁴ may all be the same, or from two to (n-1)-number may be the same and the rest different, or all n-number may be different;
X¹ to X⁴ are substituents selected from among a C₁ to C₁₀ substituted or unsubstituted alkyl group and a substituted or unsubstituted aryl group, and X¹, X², X³ and X⁴ may be bound together to form one or more rings;
Y is an atom or substituent selected from among a hydrogen atom, an oxygen atom, a C₁ to C₁₀ substituted or unsubstituted alkyl group, a substituted or unsubstituted aryl group, a C₁ to C₁₀ substituted or unsubstituted alkyloxy group, a substituted or unsubstituted aryloxy group, a mono- or di-alkylamino group, a substituted or unsubstituted arylamino group and a substituent represented by the following formula (1-2): 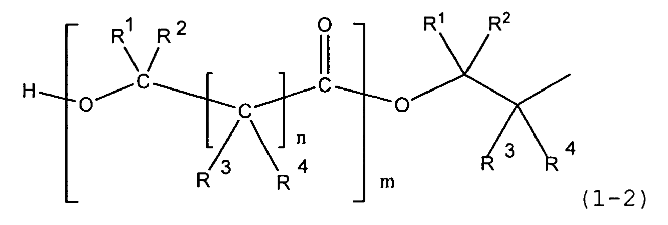 (in the formula, R¹, R², R³ and R⁴ have the same meanings as defined above; n is an integer from 1 to 7; and m is an integer from 1 to 100);
n is an integer from 1 to 7; and
m is an integer from 1 to 100.

### Detailed Description of the Invention

From the standpoint of an action of the hindered amine compound represented by the formula (1) as a light stabilizer and an anti-NOx agent being expressed well when added to a synthetic resin, each symbols in the formula are preferably as follows.

It is particularly favorable for R¹, R², R³ and R⁴ to be hydrogen atoms, it is particularly favorable for X¹ to X⁴ to be methyl groups, and it is particularly favorable for Y to be a hydrogen atom, a methyl group or a hydroxyethyl group.
n is preferably from 2 to 4, and more preferably 3 or 4. m is preferably from 1 to 80, and more preferably 2 to 50.

The hindered amine compound represented by the formula (1) can be obtained by subjecting a lactone represented by the following formula (3) to ring-opening polyaddition with an alcohol represented by the following formula (2). 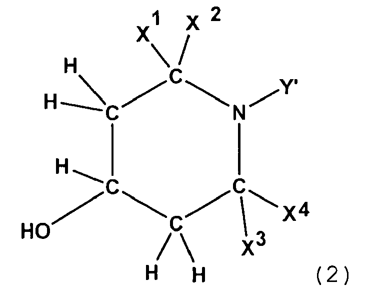 In the formula, the meanings of each symbols are as follows:
X¹ to X⁴ have the same meanings as defined above; and
Y' is an atom or substituent selected from among a hydrogen atom, an oxygen atom, a C₁ to C₁₀ substituted or unsubstituted alkyl group, a substituted or unsubstituted aryl group, a C₁ to C₁₀ substituted or unsubstituted alkyloxy group, a substituted or unsubstituted aryloxy group, a mono- or di-alkylamino group, a substituted or unsubstituted arylamino group and a substituent represented by the following formula (2-2): 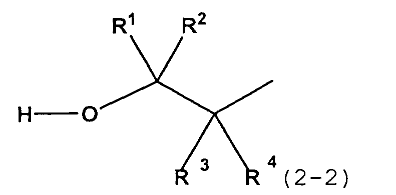 (in the formula, R¹, R², R³ and R⁴ have the same meanings as defined above.). 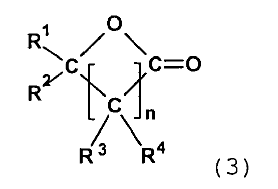 In the formula, R¹, R², R³, R⁴ and n have the same meanings as defined above.

Examples of alcohols represented by the formula (2) include 4-hydroxy-2,2,6,6-tetramethylpiperidine, 4-hydroxy-1,2,2,6,6-pentamethylpiperidine and 4-hydroxy-2,2,6,6-tetramethyl-1-(2'-hydroxyethyl)piperidine. This alcohol can be synthesized or purchased on the market.

Examples of the lactone represented by the formula (3) include ε-caprolactone, trimethyl-ε-caprolactone, monomethyl-ε-caprolactone, γ-butyrolactone and δ-valerolactone.

Examples of the catalyst used in the ring-opening polyaddition include organotitanium compounds such as tetraethyl titanate, tetrabutyl titanate or tetrapropyl titanate, organotin compounds such as stannous octylate, dibutyltin oxide, dibutyltin dilaurate or mono-n-butyltin fatty acid salt, and stannous halides such as stannous chloride, stannous bromide or stannous iodide.

The amount (based on mass) of the catalyst used is 0.1 to 10,000 ppm, and preferably 1 to 5000 ppm, with respect to the raw material used. If a catalyst is used in an amount of 0.1 ppm or more, the reaction rate of the ring-opening polyaddition of the lactone of the formula (3) will be suitably sustained, and if this amount is no more than 10,000 ppm, the reaction rate of the ring-opening polyaddition of the lactone of the formula (3) will be suitably sustained and the synthetic resin obtained using the resulting compound will not lose durability, waterproofness, or other such properties.

The reaction temperature is from 90 to 240°C, and preferably 100 to 220°C. If the reaction temperature is at least 90°C, the ring-opening polyaddition of the lactone of the formula (3) will be suitably sustained, and if the temperature is no higher than 240°C, this will prevent the depolymerization of the polylactone obtained by ring-opening polyaddition.

Conducting the reaction in an inert gas such as nitrogen gas yields better results in terms of the color of the final product and so on.

The resin composition of the present invention contains the hindered amine compound represented by the formula (1) along with a synthetic resin, and the hindered amine compound represented by the formula (1) acts mainly as a light stabilizer and an anti-NOx agent.

There are no particular restrictions on the synthetic resin used in the present invention, but when compatibility with the hindered amine compound expressed by the formula (1) is taken into account, it is especially favorable to use a thermoplastic resin.

Examples of this thermoplastic resin include polyvinyl chloride, polyvinylidene chloride, polyolefin, polycarbonate, polystyrene, acrylic resin, methacrylic resin, polyamide, polyester, acrylonitrile-butadiene-styrene (ABS) resin, thermoplastic polyurethane resin, vinyl chloride-vinylidene chloride-acrylonitrile copolymer, acrylonitrile-styrene (AS) resin, vinyl acetate resin, polyphenylene ether, polysulfone, polyether sulfone, polyether ketone and liquid crystal plastic.

Of these, it is preferable to use polyvinyl chloride, polyvinylidene chloride, polyolefin, polycarbonate, polystyrene, acrylic resin, methacrylic resin, polyamide, polyester, ABS resin, thermoplastic polyurethane resin, or the like.

In the present invention, these synthetic resins may be used singly or in mixtures of two or more types.

As for the compounding ratio of the synthetic resin and the hindered amine compound represented by the formula (1), the hindered amine compound represented by the formula (1) is preferably used in an amount of 0.01 to 20 mass parts and more preferably 0.1 to 5 mass parts, per 100 mass parts synthetic resin.

One or more known additives, such as antioxidants, light stabilizers, manufacturing stabilizers, anti-aging agents or compatibilizer can be added as needed to the resin composition of the present invention.

Examples of antioxidants include hindered phenol-based antioxidants such as 1,6-hexanediol-bis[3-(3,5-di-tert-butyl-4-hydroxyphenyl)propionate] or 3,5-di-tert-butyl-4-hydroxybenzylphosphonate diethyl ester, organosulfur-based antioxidants dilauryl 3,3'-dithiodipropionate, and phosphorus-based antioxidants such as trialkylphenyl phosphonate.

Examples of light stabilizers include hindered amine-based light stabilizers such as bis(2,2,6,6-tetramethyl-4-piperidinyl) sebacate, and nickel salt-based light stabilizers such as nickel dibutyldithiocarbamate.

Examples of manufacturing stabilizers include phosphorus-based manufacturing stabilizers such as tris(2,4-di-tert-butylphenyl) polyphosphate.

Examples of anti-aging agents include 1,1-bis(4-hydroxyphenyl)cyclohexene and N,N'-diphenyl-p-phenylenediamine.

Examples of miscibilizers include thermoplastic rubbers such as styrene-butadiene-styrene block copolymer or styrene-ethylene-butylene-styrene block copolymer.

There are no particular restrictions on the amounts of these additives used, but the amount is preferably 0.01 to 20 mass parts, and more preferably 0.1 to 10 mass parts, per 100 mass parts synthetic resin.

The resin composition of the present invention can be used in all applications in which synthetic resins are used, but can be used to particular advantage in applications with the potential for exposure to polluted air containing NOx gas, or to heat, or to sunlight or other light containing UV rays.

Specific examples include glass substitutes and glass surface coating materials, coating materials for window glass used in homes, other buildings, transportation machinery, and so on, and for natural lighting glass and light source protective glass, interior and exterior trim and paint for homes, other buildings, transportation machinery, and so on, light source members that emit UV rays, such as fluorescent lamps and mercury vapor lamps, members used with precision machinery and electronic and electrical devices, blocking members for blocking electromagnetic waves and the like emitted from various kinds of display, containers and packaging materials for foods, chemicals, drugs, and so forth, agricultural sheeting and films, agents for preventing color deterioration of printed matter, dyed goods, dyes and pigments and so forth, containers for cosmetics such as sun block cream, shampoo, hair rinse or hair conditioner, fibers and textile products used in apparel such as sportswear, stockings or caps, household interior furnishings such as curtains, carpets or wallpaper, medical devices such as plastic lenses, contact lenses or artificial eyes, optical products such as optical filters, prisms, mirrors or photographic materials, stationery such as tape or ink, signs and displays, and surface coating materials for these.

The resin composition of the present invention can be used to particular advantage in applications that require resistance to NOx.

Specific examples include elastic yarns, sheets, films, foams, paints and adhesives, and the NOx resistance is particularly pronounced when the resin composition is applied to elastic yarns or foams.

The present invention can provide a resin composition that gives a molded article having a high level of light stability, heat resistance stability and NOx resistance.

### Brief Description of Drawings

Fig. 1 is the ¹H-nuclear magnetic resonance spectrum of the compound (1) obtained in Synthesis Example 1.
Fig. 2 is the ¹³C-nuclear magnetic resonance spectrum of the compound (1) obtained in Synthesis Example 1.
Fig. 3 is the infrared absorption spectrum of the compound (1) obtained in Synthesis Example 1.
Fig. 4 is the FAB⁺-mass analysis spectrum of the compound (1) obtained in Synthesis Example 1.
Fig. 5 is a comparison of the GPC profiles for the compound (1) obtained in Synthesis Examples 1 and 2 with that of the raw material 4-hydroxy-2,2,6,6-tetramethylpiperidine.
Fig. 6 is the ¹H-nuclear magnetic resonance spectrum of the compound (1) obtained in Synthesis Example 3.

### Examples

Synthesis examples, examples and comparative examples of the hindered amine compound represented by the formula (1) will now be given in order to further clarify the present invention.

### Synthesis Example 1

81.56 g (518.6 mmol) of 4-hydroxy-2,2,6,6-tetramethylpiperidine, 118.44 g (1037.7 mmol) of ε-caprolactone and 2.0 mL (corresponds to 100 ppm of catalyst) of a 1 mass% acetone solution of mono-n-butyltin fatty acid salt (trade name: SCAT-24, made by Sankyo Organic Chemicals) were put into a glass flask equipped with a condenser pipe, a nitrogen introduction pipe, a thermometer and a stirrer.

With the reaction temperature held at 160°C, the ε-caprolactone concentration in the reaction solution was measured by gas chromatography, and 20 g of xylene was added at the point when the ε-caprolactone concentration hit 30 mass% or less. 15 hours later, the ε-caprolactone concentration in the reaction solution was measured by gas chromatography and found to be 0.25 mass%, so the reaction was halted.

This reaction product was in the form of a pale brown wax at room temperature, and the results of ¹H-nuclear magnetic resonance spectral analysis revealed it to have a structure in which an average of 3.9 ε-caprolactone groups had been added to 66.8% of the hydroxyl group site of the 4-hydroxy-2,2,6,6-tetramethylpiperidine skeleton.

The results of GPC analysis with a tetrahydrofuran eluant revealed the number average molecular weight (Mn) to be 456, the weight average molecular weight (Mw) to be 754 (both calculated as polyethylene glycol), and the Mw/Mn ratio to be 1.65.

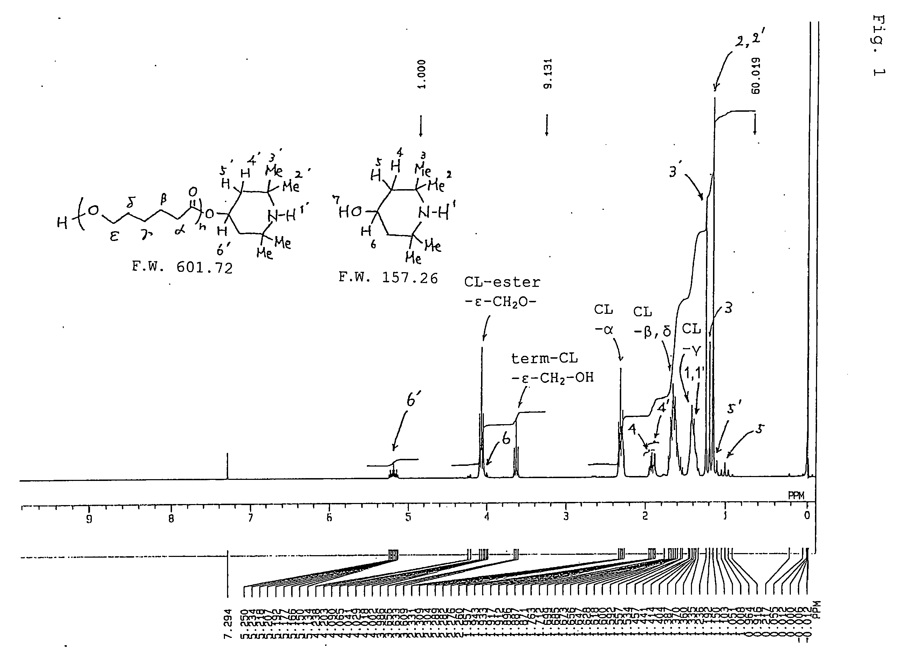

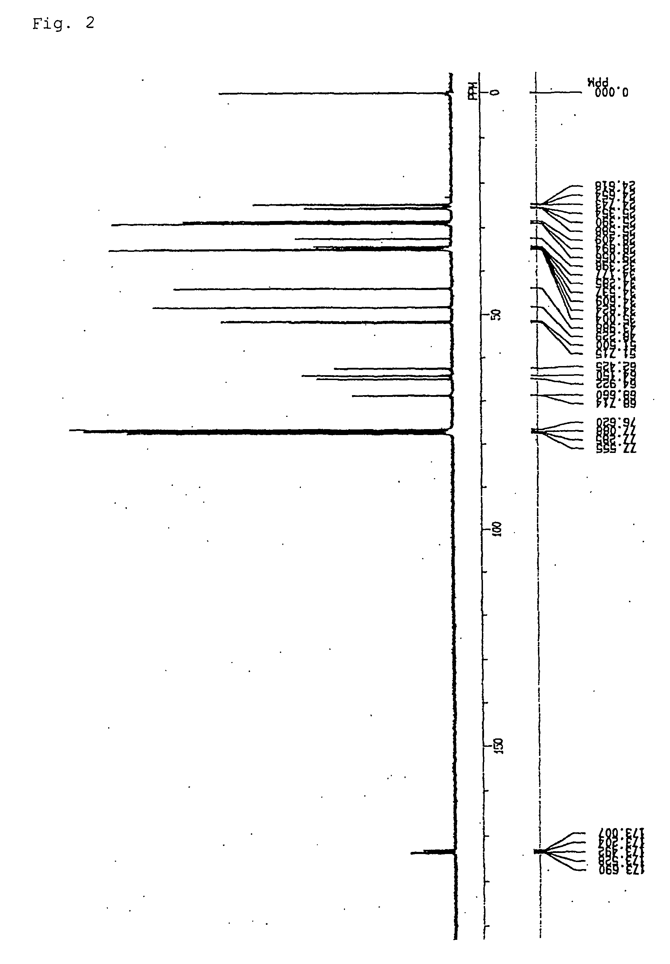

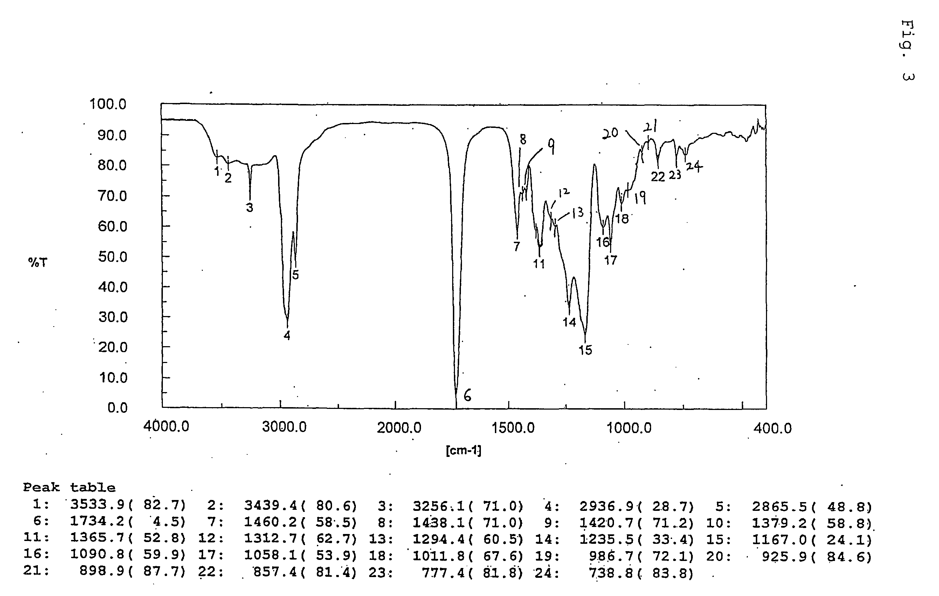

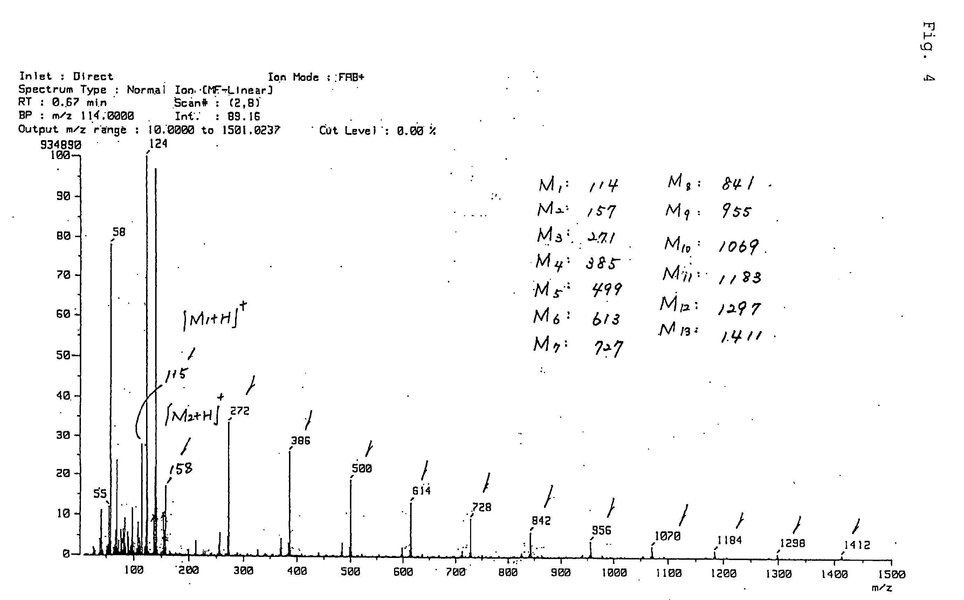

The ¹H-nuclear magnetic resonance spectrum of the compound (1) obtained above is shown in Fig. 1, the ¹³C-nuclear magnetic resonance spectrum in Fig. 2, the infrared absorption spectrum measurement results in Fig. 3, and the FAB⁺-mass analysis spectrum in Fig. 4.

### Synthesis Example 2

43.19 g (274.6 mmol) of 4-hydroxy-2,2,6,6-tetramethylpiperidine, 156.81 g (1373.8 mmol) of ε-caprolactone and 1.0 mL (corresponds to 50 ppm of catalyst) of a 1 mass% acetone solution of mono-n-butyltin fatty acid salt (trade name: SCAT-24, made by Sankyo Organic Chemicals) were added to a glass flask equipped with a condenser pipe, a nitrogen introduction pipe, a thermometer and a stirrer.

With the reaction temperature held at 160°C, the ε-caprolactone concentration in the reaction solution was measured by gas chromatography, and 20 g of xylene was added at the point when the ε-caprolactone concentration hit 30 mass% or less. 13 hours later, the ε-caprolactone concentration in the reaction solution was measured by gas chromatography and found to be 0.73 mass%, so the reaction was halted.

This reaction product was in the form of a pale brown wax at room temperature, and the results of ¹H-nuclear magnetic resonance spectral analysis revealed it to have a structure in which an average of 6.7 ε-caprolactone groups had been added to 75.1% of the hydroxyl group site of the 4-hydroxy-2,2,6,6-tetramethylpiperidine skeleton.

The results of GPC analysis with a tetrahydrofuran eluant revealed the number average molecular weight (Mn) to be 810, the weight average molecular weight (Mw) to be 1258 (both calculated as polyethylene glycol), and the Mw/Mn ratio to be 1.55.

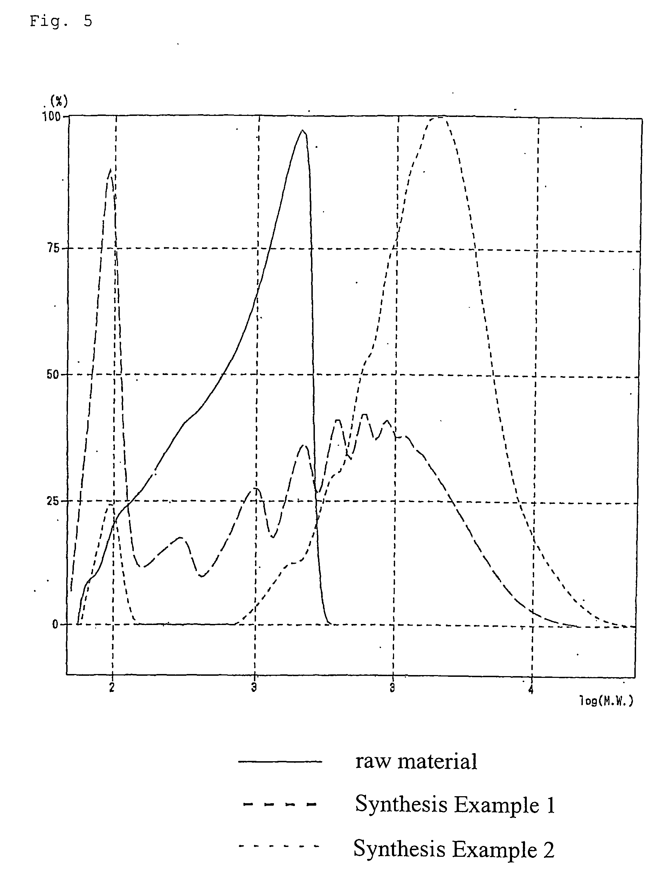

The GPC profile of the hindered amine compound of the formula (1) obtained above is shown in Fig. 5, and is compared with the hindered amine compound of the formula (1) obtained in Synthesis Example 1, and with the raw material 4-hydroxy-2,2,6,6-tetramethylpiperidine.

### Synthesis Example 3

99.32 g (314.1 mmol) of 4-hydroxy-2,2,6,6-tetramethylpiperidine, 250.68 g (2196 mmol) of ε-caprolactone and 1.5 mL (corresponds to 50 ppm of catalyst) of a 1 mass% acetone solution of mono-n-butyltin fatty acid salt (trade name: SCAT-24, made by Sankyo Organic Chemicals) were added to a glass flask equipped with a condenser pipe, a nitrogen introduction pipe, a thermometer and a stirrer.

With the reaction temperature held at 160°C, the ε-caprolactone concentration in the reaction solution was measured by gas chromatography, and 52.9 g of xylene was added at the point when the ε-caprolactone concentration hit 30 mass% or less. 13 hours later, the ε-caprolactone concentration in the reaction solution was measured by gas chromatography and found to be 0.07 mass%, so the reaction was halted.

This reaction product was in the form of a pale brown wax at room temperature, and the results of ¹H-nuclear magnetic resonance spectral analysis revealed it to have a structure in which an average of 9.3 ε-caprolactone groups had been added to 85% of the hydroxyl group site of the 4-hydroxy-2,2,6,6-tetramethylpiperidine skeleton.

The results of GPC analysis with a tetrahydrofuran eluant revealed the number average molecular weight (Mn) to be 1700, the weight average molecular weight (Mw) to be 2270 (both calculated as polyethylene glycol), and the Mw/Mn ratio to be 1.34. Synthesis Example 4

36.31 g (231.7 mmol) of 4-hydroxy-2,2,6,6-tetramethylpiperidine, 263.69 g (2310 mmol) of ε-caprolactone and 1.5 mL (corresponds to 50 ppm of catalyst) of a 1 mass% acetone solution of mono-n-butyltin fatty acid salt (trade name: SCAT-24, made by Sankyo Organic Chemicals) were added to a glass flask equipped with a condenser pipe, a nitrogen introduction pipe, a thermometer and a stirrer.

With the reaction temperature held at 160°C, the ε-caprolactone concentration in the reaction solution was measured by gas chromatography, and 52.9 g of xylene was added at the point when the ε-caprolactone concentration hit 30 mass% or less. 13 hours later, the ε-caprolactone concentration in the reaction solution was measured by gas chromatography and found to be 0.08 mass%, so the reaction was halted.

This reaction product was in the form of a pale brown wax at room temperature, and the results of ¹H-nuclear magnetic resonance spectral analysis revealed it to have a structure in which an average of 12.1 ε-caprolactone groups had been added to 88% of the hydroxyl group site of the 4-hydroxy-2,2,6,6-tetramethylpiperidine skeleton.

The results of GPC analysis with a tetrahydrofuran eluant revealed the number average molecular weight (Mn) to be 1860, the weight average molecular weight (Mw) to be 2800 (both calculated as polyethylene glycol), and the Mw/Mn ratio to be 1.50. Synthesis Example 5

28.74 g (182.8 mmol) of 4-hydroxy-2,2,6,6-tetramethylpiperidine, 271.26 g (2376.6 mmol) of ε-caprolactone and 1.5 mL (corresponds to 50 ppm of catalyst) of a 1 mass% acetone solution of mono-n-butyltin fatty acid salt (trade name: SCAT-24, made by Sankyo Organic Chemicals) were added to the same apparatus as in Synthesis Example 1.

With the reaction temperature held at 150°C, the ε-caprolactone concentration in the reaction solution after 10 hours was measured by gas chromatography and found to be 0.49 mass%, so the reaction was halted. This reaction product was in the form of a pale brown wax at room temperature, and the results of ¹H-nuclear magnetic resonance spectral analysis revealed it to have a structure in which an average of 14.3 ε-caprolactone groups had been added to 93% of the hydroxyl group site of the 4-hydroxy-2,2,6,6-tetramethylpiperidine skeleton.

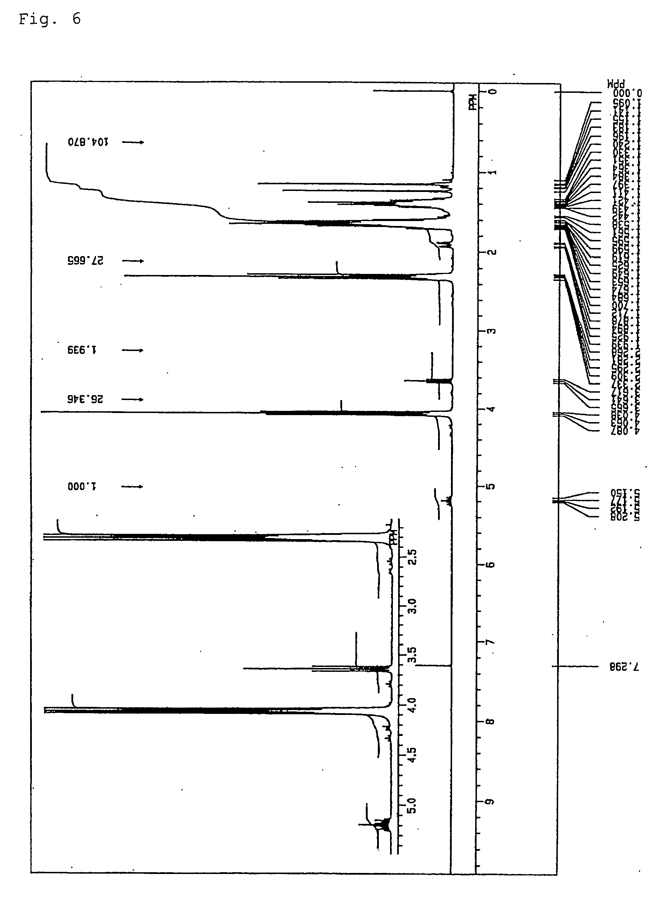

The ¹H-nuclear magnetic resonance spectrum of the compound (1) obtained in this manner is shown in Fig. 6. The results of GPC analysis with a tetrahydrofuran eluant revealed the number average molecular weight (Mn) to be 2250, the weight average molecular weight (Mw) to be 3850 (both calculated as polyethylene glycol), and the Mw/Mn ratio to be 1.71.

### Synthesis Example 6

15.73 g (100 mmol) of 4-hydroxy-2,2,6,6-tetramethylpiperidine, 184.27 g (1614.4 mmol) of ε-caprolactone, and 1 mL (corresponds to 50 ppm of catalyst) of a 10 mass% acetone solution of mono-n-butyltin fatty acid salt (trade name: SCAT-24, made by Sankyo Organic Chemicals) were added to the same apparatus as in Synthesis Example 1.

With the reaction temperature held at 150°C, the ε-caprolactone concentration in the reaction solution after 15 hours was measured by gas chromatography and found to be 0.30 mass%, so the reaction was halted. This reaction product was in the form of a pale brown wax at room temperature.

### Synthesis Example 7

20.13 g (100 mmol) of N-(2-hydroxyethyl)-4-hydroxy-2,2,6,6-tetramethylpiperidine, 179.87 g (1575.8 mmol) of ε-caprolactone, and 1 mL (corresponds to 50 ppm of catalyst) of a 10 mass% acetone solution of mono-n-butyltin fatty acid salt (trade name: SCAT-24, made by Sankyo Organic Chemicals) were added to the same apparatus as in Synthesis Example 1.

With the reaction temperature held at 150°C, the ε-caprolactone concentration in the reaction solution after 15 hours was measured by gas chromatography and found to be 0.33 mass%, so the reaction was halted. This reaction product was in the form of a pale brown wax at room temperature.

### Synthesis Example 8

17.13 g (100 mmol) of N-methyl-4-hydroxy-2,2,6,6-tetramethylpiperidine, 182.87 g (1602.1 mmol) of ε-caprolactone and 1 mL (corresponds to 50 ppm of catalyst) of a 10 mass% acetone solution of mono-n-butyltin fatty acid salt (trade name: SCAT-24, made by Sankyo Organic Chemicals) were added to the same apparatus as in Synthesis Example 1.

With the reaction temperature held at 150°C, the ε-caprolactone concentration in the reaction solution after 15 hours was measured by gas chromatography and found to be 0.36 mass%, so the reaction was halted. This reaction product was in the form of a pale brown wax at room temperature.

### Example 1 and Comparative Example 1

Each 0.25 mass part of the hindered amine compound obtained in each of the above synthesis examples and a UV absorbent (trade name: Tinuvin 326, made by Ciba Specialty Chemicals) were mixed with 100 mass parts of polypropylene. This mixture was kneaded for 10 minutes at 200°C in a Laboplast mill, then press-molded for 6 minutes at 260°C to obtain a test piece in the form of a film with a thickness of 0.1 mm.

This 0.1 mm-thick film was subjected to an exposure test with a sunshine weather-o-meter, and after exposure for the time listed in Table 1, the film was subjected to a tensile strength test and its tensile elongation at break was measured, and the half-life time (HLT; time required for elongation to decrease to 50% of its initial value) was evaluated as degradation. The improvement coefficients given in Table 1 were calculated from the following formula. The test results are given in Table 1.

Improvement coefficient = (HLT when light stabilizer is added)/(HTL when light stabilizer is not added)
(1) Exposure test machine: Sunshine Weather-o-meter WEL-SUN-HC (made by Suga Test Instruments); no spray; temperature: black panel 63 ± 3°C
(2) Tensile strength test machine: Shimadzu Autograph AGS-500D (supplement), made by Shimadzu Corporation

Test conditions: cross head speed: 120 mm/min; chuck gap: 25 mm

**Table 1**

| | Additive | Light resistance (HLT/hour) | Improvement coefficient |
|---|---|---|---|
| Comparative Example 1-1 | none added | 180 | 1.0 |
| Comparative Example 1-2 | Tinuvin-326 | 310 | 1.7 |
| Example 1-1 | Synthesis Example 1 | 720 | 4.0 |
| Example 1-2 | Synthesis Example 2 | 570 | 3.2 |
| Example 1-3 | Synthesis Example 3 | 440 | 2.4 |
| Example 1-4 | Synthesis Example 4 | 370 | 2.0 |
| Example 1-5 | Synthesis Example 5 | 320 | 1.8 |

It can be seen from Table 1 that the products of the present invention have a superior effect in terms of improving light resistance stability as compared to the conventional products.

### Example 2 and Comparative Example 2

Each 0.25 mass part of the hindered amine compound obtained in each of the above synthesis examples and a UV absorbent (trade name: Tinuvin 326, made by Ciba Specialty Chemicals) were mixed with 100 mass parts of polypropylene. This mixture was kneaded for 10 minutes at 200°C in a Laboplast mill, then press-molded for 6 minutes at 260°C to obtain a test piece in the form of a sheet with a thickness of 0.5 mm.

This 0.5 mm-thick sheet was placed in a 150°C air oven to examined how many days it took for the sheet to become brittle. The test results are given in Table 2.

**Table 2**

| | Additive | Days at 150°C until embrittlement |
|---|---|---|
| Comparative Example 2-1 | none added | 4 |
| Comparative Example 2-2 | Tinuvin-326 | 4 |
| Example 2-1 | Synthesis Example 1 | 6 |
| Example 2-2 | Synthesis Example 2 | 6 |
| Example 2-3 | Synthesis Example 3 | 6 |
| Example 2-4 | Synthesis Example 4 | 6 |
| Example 2-5 | Synthesis Example 5 | 6 6 |

It can be seen from Table 2 that the products of the present invention have a superior effect in terms of improving heat resistance stability as compared to the conventional products.

### Example 3 and Comparative Example 3

(1) Production of film
   2000 g of polytetramethylene glycol (PTG2000, made by Mitsubishi Chemical, molecular weight of 2000), 180 g of 1,4-butanediol (made by Mitsubishi Chemical) and 5867.6 g of dimethylformamide were put into a glass flask equipped with a condenser pipe, a nitrogen introduction pipe, a thermometer and a stirrer.
   With the reaction temperature set at 80°C, 787.5 g of diphenylmethane diisocyanate (MDI, made by Nippon Polyurethane Kogyo) was gradually added thereto, and the reaction was continued until the solution viscosity reached 68,000 mPa·s (25°C), to give a polyurethane solution with a solids concentration of 30 mass%.
   The various additives listed in Tables 3 and 4 were added to this polyurethane solution and mixed under stirring at room temperature. The resulting solution was used to coat non-stick paper such that the film thickness after drying would be approximately 150 µm, and the coating was forcibly dried for about 2 hours at 120°C to obtain a polyurethane film. Details of the various additives used here are given below.
   Ethanox 330: antioxidant made by Albemarle
   PCL UVA103: UV absorbent made by Daicel Chemical Industries
   Tinuvin-234: UV absorbent made by Ciba Specialty Chemicals
   Cyasorb UV-1164: UV absorbent made by Sun Chemical
   HN 130: anti-NOx agent made by Japan Hydrazine hindered amine compounds of Synthesis Examples 1 to 5
(2) A NOx gas was prepared using the method and apparatus set forth in JIS L 0855 (1979), and a NOx resistance test was conducted using an exposure tester made by Suga Test Instruments. The exposure test was conducted for 16 hours at a NOx concentration (mass standard) of 1000 ppm or for 24 hours at 2000 ppm, and the NOx resistance was evaluated from the YI value (initial value) prior to the test, the YI value after the test, and the color difference (ΔE*). The smaller the ΔE* value, the better the NOx resistance.

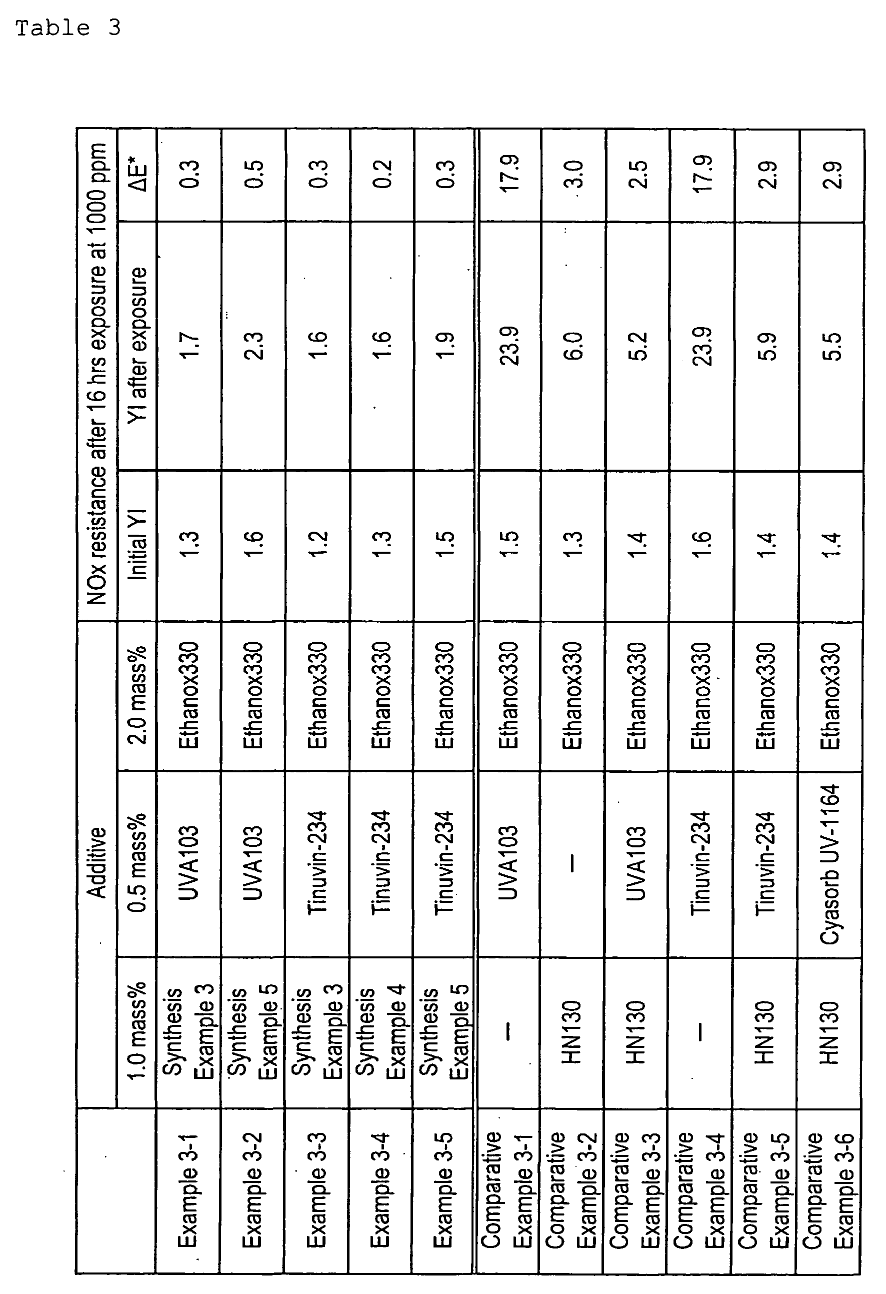

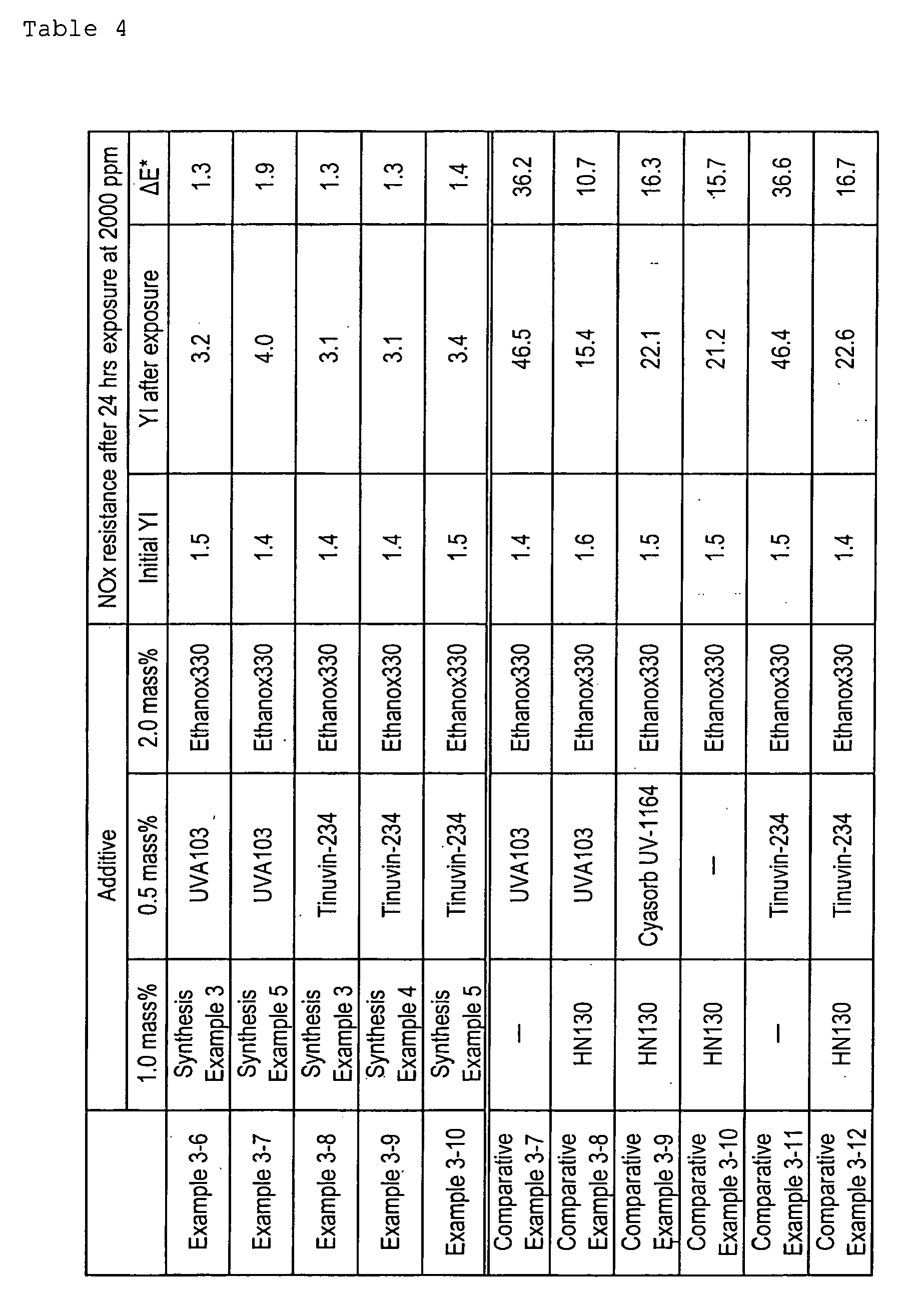

It can be seen from Tables 3 and 4 that the products of the present invention have a superior effect in terms of improving NOx resistance as compared to the conventional products.

## Claims

1. A hindered amine compound represented by the formula (1): 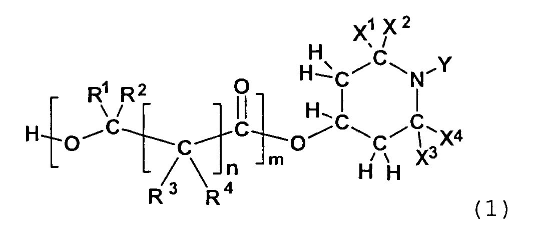 in the formula, the meanings of each symbols are as follows:
R¹, R², R³ and R⁴ are atoms or substituents selected from among a hydrogen atom, a C₁ to C₁₀ substituted or unsubstituted alkyl group and a substituted or unsubstituted aryl group, and R¹, R², R³ and R⁴ may be bound together to form one or more rings;
R³ and R⁴ may be the same as or different from each other, n-number of R³ and R⁴ may all be the same, or from two to (n-1)-number may be the same and the rest different, or all n-number may be different;
X¹ to X⁴ are substituents selected from among a C₁ to C₁₀ substituted or unsubstituted alkyl group and a substituted or unsubstituted aryl group, and X¹, X², X³ and X⁴ may be bound together to form one or more rings;
Y is an atom or substituent selected from among a hydrogen atom, an oxygen atom, a C₁ to C₁₀ substituted or unsubstituted alkyl group, a substituted or unsubstituted aryl group, a C₁ to C₁₀ substituted or unsubstituted alkyloxy group, a substituted or unsubstituted aryloxy group, a mono- or di-alkylamino group, a substituted or unsubstituted arylamino group and a substituent represented by the following formula (1-2): 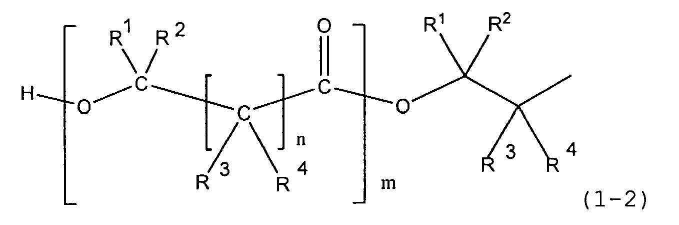 (in the formula, R¹, R², R³ and R⁴ have the same meanings as defined above; n is an integer from 1 to 7; and m is an integer from 1 to 100);
n is an integer from 1 to 7; and
m is an integer from 1 to 100.

2. A method for manufacturing the hindered amine compound represented by the formula (1) according to Claim 1, which comprises the step of subjecting a lactone represented by the formula (3): 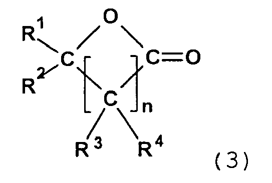 (in the formula, R¹, R², R³, R⁴ and n have the same meanings as defined above) to ring-opening polyaddition with an alcohol represented by the formula (2): 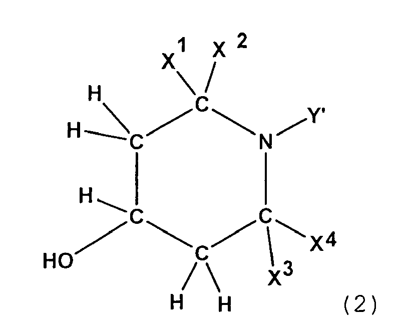 (in the formula, the meanings of each symbols are as follows:
X¹ to X⁴ have the same meanings as defined above; and
Y' is an atom or substituent selected from among a hydrogen atom, an oxygen atom, a C₁ to C₁₀ substituted or unsubstituted alkyl group, a substituted or unsubstituted aryl group, a C₁ to C₁₀ substituted or unsubstituted alkyloxy group, a substituted or unsubstituted aryloxy group, a mono- or di-alkylamino group, a substituted or unsubstituted arylamino group and a substituent represented by the following formula (2-2): 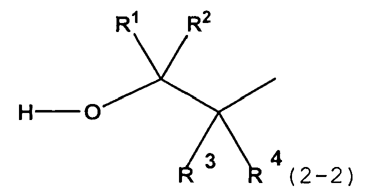 (in the formula, R¹, R², R³ and R⁴ have the same meanings as defined above)).

3. A resin composition containing a synthetic resin and a hindered amine compound represented by the formula (1) .

4. The resin composition according to Claim 3, wherein the synthetic resin is at least one selected from the group consisting of polyvinyl chloride, polyvinylidene chloride, polyolefin, polycarbonate, polystyrene, acrylic resin, methacrylic resin, polyamide, polyester, acrylonitrile-butadiene-styrene resin and thermoplastic polyurethane resin.

5. A molded article obtained from the resin composition according to Claim 3 or 4.
